# NEW EUROPEAN PATENT SPECIFICATION

(11) **EP 1 464 677 B2**
(45) Date of publication and mention of the opposition decision: **12.10.2016**
(45) Mention of the grant of the patent: 14.04.2010
(21) Application number: 04076881.4
(22) Date of filing: 03.10.1997
(51) Int. Cl.: C07H 21/00, C08J 7/04, G01N 33/543

(54) **Method for producing low binding surfaces**
Verfahren zum Herstellen von Oberflächen mit niedriger Bindungsaffinität
Procédé de production de surfaces à faible liaison

(30) Priority: 24.10.1996 US 29009 P
(43) Date of publication of application: 06.10.2004
(62) Divisional of application: 97945529.2
(73) Proprietor: Corning Incorporated, Corning, NY 14831 (US)
(72) Inventor: Bookbinder, Dana C., Corning, NY 14830 (US); Fewkes Jr., Edward J., Horseheads, NY 14845 (US); Griffin, James A., Corning, NY 14830 (US); Smith, Frances M., Horseheads, NY 14845 (US); Tennent, David L., Campbell, NY 14821 (US)
(74) Representative: Kingsbury, Oliver William

(56) References cited:
- EP-A- 0 511 127
- EP-A- 0 524 404
- EP-A2- 0 735 089
- GB-A- 2 078 760
- US-A- 4 800 115
- DATABASE CHEMABS CHEMICAL ABSTRACTS SERVICE, COLUMBUS, OHIO, US; VOLUME 80, NO. 24 17 June 1974 (1974-06-17), XP002293974 Database accession no. 80:134256 & JP 48 024016 B (CHIBA FINE CHEMICALS CO., LTD.) 18 July 1973 (1973-07-18)
- JP H08258881 AND THE ENGLISH TRANSLATION
- WILLIAMS J.B. ET AL: 'Optimization of Antistatic Additives in Polypropylene' JOURNAL OF VINYL ADDITIVE TECHNOLOGY vol. 1, no. 4, December 1995, pages 282 - 285
- RABINOW B.E. ET AL: 'Biomaterials with permanent hydrophilic surfaces and low protein adsorption properties' J. BIOMATER. SCI. POLYMER EDN. vol. 6, no. 1, 1994, pages 91 - 109
- MORRA M. ET AL: 'Surface Modification of Blood Contacting Polymers by Poly(ethyleneoxide)' CLINICAL MATERIALS vol. 14, 1993, pages 255 - 265
- ANDRADE J.D. ET AL: 'Proteins at Interfaces: Principles, Multivariate Aspects, Protein Resistant Surfaces, and Direct Imaging and Manipulation of Adsorbed Proteins' CLINICAL MATERIALS vol. 11, 1992, pages 67 - 84
- Cole-Parmer catalog - printouts from the website
- Professional Plastics - Laboratory supplies - printouts from the website
- National Organic Standards Board Technical Advisory Panel Review - data sheets for glycerol monooleate
- The Pharmaceutics and Compounding Laboratory - data sheet for emulsifiers and their HLB Values
- BASF - Product data sheet for Pluronic F-127
- Product Technical Information about polyproylene-homopolymer - data sheet from INEOS
- Ciba data sheet for Atmer 7306

## Description

### FIELD OF THE INVENTION

This invention relates to reducing the binding of organic materials (e.g., peptides, proteins, nucleic acids, and cells) to hydrophobic surfaces (e.g., polymeric surfaces). More particularly the invention relates to the use of a polymer blend for the manufacture of labware having such low binding surfaces.

### BACKGROUND OF THE INVENTION

Biological materials such as peptides, proteins, nucleic acids, and cells are often stored or transferred in containers such as centrifuge tubes and pipettes made of plastic or other hydrophobic materials. It is a common observation that biological compounds adsorb/bind to the surfaces of such containers. This is also true for organic materials which exhibit some hydrophobicity in an aqueous solution, e.g., acridinium compounds, PCBs, etc.

For many applications, such binding is undesirable. For example, the binding results in the loss of valuable materials, such as, enzymes and antibodies, and can result in variations in the dispensing of organic materials, especially when small volumes are involved. The binding of proteins, cells, and platelets to hydrophobic surfaces is also of concern in a variety of blood handling procedures.

As a result of these considerations, extensive efforts have been made to provide methods for reducing the binding of proteins and other organic compounds to hydrophobic surfaces. Examples of the approaches which have been considered can be found in Caldwell et al., U.S. Patent No. 5,516,703; Ding *et al*., International Application Publication W094/03544; Amiji et al., Biomaterials, 13:682-692, 1992; J. Andrade, "Principles of Protein Adsorption" in Surface and Interfacial Aspects of Biomedical Polymers, J. Andrade, editor, Volume 2, Plenum Press, New York, 1-80, 1985; Lee et al., Polymeric Mater. Sci Eng., 57:613-617, 1987; Lee et al., Journal of Biomedical Materials Research, 23:351-368, 1989; Lee et al., Biomaterials, 11:455-464, 1990; Lee et al., Prog. Polym. Sci., 20:1043-1079, 1995; Merrill et al., ASAIO Journal, 6:60-64, 1983; Okano et al., Journal of Biomedical Materials Research, 20:1035-1047, 1986; Okkema et al., J. Biomater. Sci. Polymer Edn., 1:43-62, 1989; Owens et al., Journal of Cell Science, 87:667-675, 1987; Rabinow et al., J. Biomater. Sci. Polymer Edn., 6:91-109, 1994; Schroen et al., Journal of Membrane Science, 80:265-274, 1993; Sheu et al., J. Adhesion Sci. Technol., 6:995-1009, 1992; Shimada et al., Polymer Journal, 15:649-656, 1983; and Thurow et al., Diabetologia, 27:212-218, 1984.

The criteria which a successful technique for producing a low binding surface should satisfy include: 1) a sufficiently low level of binding; 2) substantial permanence; 3) ease of use; and 4) low cost. It is the goal of the present invention to provide labware with low binding surfaces which satisfy all of these criteria.

EP-A-0 511 127 relates to a packaging film made from butadienestyrene copolymer to which is added glycerol monostearate.

EP-A-0 524 404 relates to a resin composition for the manufacture of packaging materials for packaging photosensitive materials. The composition comprises a thermo-plastic resin, at least one lubricant, an antioxidant and an organic nucleating agent and a so-called "drip-proofing agent" which inhibits and equalises the bleeding out of the lubricant, antioxidant and organic nucleating agent. The "drip-proofing agent" may be selected from a wide range of surface active agents including sorbitan monolaurate and sorbitan monostearate.

JP-48024016 (Chemical abstracts 80: 134256) relates to antifogging polyolefin films containing a surfactant such as glycerol monostearate.

### SUMMARY OF THE INVENTION

The present invention provides the use of a product made from a polymer blend comprising:
a) a non-water soluble non-ionic surfactant having a hydrophilic-lipophilic balance number which is less than or equal to 10 and having at least one hydrophilic element which can extend into aqueous solution selected from alkyl alcohol ethoxylates, alkyl ester ethoxylates, sorbitol alkyl esters, ethylene oxide/propylene oxide block co-polymers and polypropylene oxide; and
b) a matrix polymer, wherein said product is capable of reducing the ability of an organic material in an aqueous solution to bind to its surface, for the manufacture of labware.

The specific materials employed in the invention are non-ionic surfactants which have a hydrophilic element which can extend into an aqueous solution, e.g., a hydrophilic end group, and have a hydrophilic-lipophilic balance number (HLB number) which is less than or equal to 10. The term "non-ionic surfactant" is used herein in accordance with its classical definition as a molecule containing two structurally dissimilar groups having different solubilities in an aqueous solution. See Kirk-Othmer Encyclopedia of Chemical Technology, Third Edition, Volume 22, page 332, John Wiley & Sons, New York, New York, 1983.

It has been found according to the present invention, that surfactants having an HLB number of less than or equal to 10 can be blended in with a number of base polymer thermoplastics prior to molding. A sufficient number of low HLB number molecules migrate to the surface during the molding process, a process called "blooming", to yield a low binding surface.

The process employed in this embodiment comprises the steps of thoroughly mixing the non-water soluble non-ionic surfactant with a matrix polymer into a blend, melting the blend, and exposing the blend to sheer conditions such that the non-ionic surfactant will move to the surface of the polymer substrate through sheer. For example, an extruder may be used to blend the materials and an injection molding machine may be used to transform the polymer blend into a finished part. Once the non-ionic surfactant has migrated to the surface of the polymer, the hydrophilic element of the surfactant molecule extends from the polymer surface into an aqueous solution. The resultant product exhibits the non-binding characteristics consistent with products that have been coated with the surfactant. An advantage of using the blending process lies in the elimination of the drying step and the coating step, thereby further aiding in cost reduction.

Ding *et al.* discloses the use of polymer blends containing watersoluble polymers for creating a low protein binding surface on a substrate polymer. However, Ding *et. al.* does not disclose the addition of non-ionic, non-water soluble surfactants having an HLB number less than or equal to 10, to a polymer blend.

A particularly advantageous application of the invention is in the production of labware having protein resistant surfaces. Examples of the types of products which can be provided with low-binding surfaces in accordance with the invention include containers of all shapes, sizes, and descriptions, multiwell strips, pipettes, pipette tips, membranes, reagent reservoirs, storage vessels, tubing and the like. Once provided with a low binding surface, these products can be sterilized using conventional techniques such as gamma-ray sterilization.

### DESCRIPTION OF THE PREFERRED EMBODIMENTS

As discussed above, the present invention relates to the creation of low-binding surfaces on hydrophobic substrates through the use of non-ionic surfactants having a HLB number less than or equal to 10 and a hydrophilic element which can extend into an aqueous solution. Discussions of HLB numbers and how they are determined for specific surfactants can be found in, for example, the publication of ICI Surfactants entitled The HLB System and, in particular, in Chapter 7 of that publication entitled "How to Determine HLB of an Emulsifier" (ICI Americas, Inc., Wilmington, Delaware, 1992).

The non-ionic surfactant used in the practice of the invention needs to have a hydrophilic element which can extend into an aqueous solution so as to provide the requisite low-binding surface. Although not wishing to be bound by any particular theory of operation, it is believed that such a hydrophilic element when hydrated and extending away from a hydrophobic surface provides an aqueous boundary layer which cannot be readily penetrated by molecules having hydrophobic regions, e.g., proteins, thus preventing such molecules from binding to the hydrophobic surface. In many cases, the non-ionic surfactant molecules used in the practice of the invention will have a central hydrophobic region connected at each end to a hydrophilic element which can extend into an aqueous solution. In other cases, the molecules will have a hydrophobic region connected on only one end to a hydrophilic element. Surfactant molecules having other configurations can, of course, be used if desired provided they have at least one hydrophilic element which can extend into an aqueous solution. It should be noted that in the limit, the hydrophilic element can be as simple as a hydroxyl group.

The presence of the hydrophilic element or elements means that the surfactant molecules will normally have a HLB number greater than zero, i.e., they will have some hydrophilic character. (Note that in the case of polypropylene oxide, the HLB number is in effect close to zero, i.e., it is less than 0.5.) However, since the HLB number must be less than or equal to 10 to achieve a substantially permanent, low-binding surface, this hydrophilic character is significantly less than the molecule's lipophilic character. Generally, non-ionic surfactants having HLB numbers less than about 2.5 are preferred for the practice of the invention.

A variety of non-ionic surfactants now known or subsequently developed can be used in the practice of the invention. Suitable surfactants are alkyl alcohol ethyoxylates, alkyl ester ethyoxylates, sorbitol alkyl esters, and ethylene oxide/propylene oxide block co-polymers. As discussed above, polypropylene oxide can also be used in the practice of the invention. Preferably, the polypropylene oxide will have an average molecular weight in the range of from about 1,000 to about 15,000. Derivatives of polypropylene oxide, such as branched and star polymers, can also be used in the practice of the invention.

These and other suitable surfactants can be obtained from a variety of manufacturers including ICI Americas, Inc., Wilmington, Delaware; BASF Corp., Parsippany, New Jersey; Witco Corp., Greenwich, Connecticut; and the Henkel Corporation Ambler, Pennsylvania. If desired, mixtures of non-ionic surfactants can be used in the practice of the invention, provided each surfactant used in the mixture has a HLB number less than 10. Lists of various commercially available non-ionic surfactants can be found in McCutcheon's Emulsifiers and Detergents, North American edition, The Manufacturing Confectioner Publishing Co., Glen Rock, New Jersey, 1995. A preferred non-ionic surfactant for use in the present invention is Pluronic® L-121.

A variety of hydrophobic surfaces can be made low-binding in accordance with the invention.

Examples of the types of polymeric surfaces which can benefit from the invention include those comprising or composed of polystyrene, polypropylene, polymethyl methacrylate, polyvinyl chloride, polymethyl pentene, polyethylene, polycarbonate, polysulfone, polystyrene copolymers (e.g., SAN and ABS), polypropylene copolymers, fluoropolymers, polyamides, silicones, and elastomers, including silicone, hydrocarbon, and fluorocarbon elastomers.

In producing non-binding surfaces by adding the non-ionic surfactant to the blend prior to molding, the ionic surfactant molecules were blended at 5% into a base polymer. The required amount of non-ionic surfactant to be added to the matrix polymer may vary depending on the molecule, anywhere within a preferred range of 0.10 - 10.0 % (weight/volume), and more preferably between 0.5 - 5.0%. It should be noted that other additives such as dyes, pigments, stabilizers, impact modifiers and the like may be added to the blend to create a finished product having certain desired characteristics.

Without intending to limit it in any manner, the present invention will be more fully described by the following example.

### DETERMINATION OF REDUCED PROTEIN BINDING BY COLLOIDAL GOLD STAINING

Protein binding was determined using a colloidal gold staining procedure. In accordance with this procedure, colloidal gold becomes electrostatically attached to bound protein and is detected by measuring absorbance at 550 nanometers. Bio-Rad Laboratories' Colloidal Gold Total Protein Stain was used for this purpose, with absorbance being measured with a Cambridge Technologies Plate Reader (No. 7520).

As generally understood in the art, the upper limit for a low binding surface is about 50-80 ng/cm². For comparison, medium binding, such as that exhibited by untreated polystyrene or polypropylene, is around 200-400 ng/cm², and high binding, such as that exhibited by a polystyrene or polypropylene surface which has been plasma oxidized and gamma radiation sterilized, is around 400-800 ng/cm².

Plates are incubated with IgG as follows: 0.1 ml of Horse IgG (Pierce Labs) at a working concentration of 10 µg/ml in 0.1 M NaHCO₃ buffer (pH9.4) are added to each well, incubated 30 minutes on a rocker table, and rinsed with dl H₂O. The plates are gold stained overnight (0.3ml/well), rinsed in dl H₂O, air dried, and absorbance measured at 550nm.

### Example

In order to demonstrate the binding effect of polymer surfaces molded from a blend containing a non-ionic surfactant, several low binding molecules and matrix polymers were blended and subsequently molded. Low binding non-ionic surfactant molecules were blended at 5% (except as noted in the following Table) into a base polymer using a twin screw extruder, then injection molded into the shape of 35 mm petri dishes. Proteins tested were BSA protein (Bovine Serum Albumin, Fraction V, Sigma) and Fetal Bovine Serum (FBS). Protein binding was determined using a colloidal gold staining procedure as described above. In the case of the polypropylene and EVA blends, a silver enhancer, was used (Sigma Chem. Co., St, Louis, MO, Kit # SE-100).

It was discovered that while not each non-ionic surfactant having the requisite low HLB number (≤ 10) was able to migrate to the surface of each base polymer in order to create a noticeable non-binding effect, a sufficient number of blends exhibited the desired non-binding characteristics to indicate that, in those instances, much of the low HLB non-ionic surfactant had indeed bloomed to the polymer surface. The reason that some molecules having the requisite HLB number do not bloom to the surface of some polymers while others do, is not fully understood. However, such an understanding is not necessary in order to practice this embodiment of the invention.

The base or matrix polymer used for the blend may be selected from the group including ethylene vinyl acetate, polypropylene, polyolefin, polyvinylchloride, polystyrene, polystyrene-butadiene copolymer, polycarbonate, polyacrylate, polyamide, and copolymers thereof, polyurethane, polyester and copolymers thereof, and fluoropolymers.

As shown in the following Table, non ionic surfactants with HLB numbers less than 10, such as sorbitol mono-oleate (HLB=4.3), PEO stearyl alcohol (HLB=4.7) and PEO oleyl ether (HLB=4.9) in polypropylene or EVA resulted in no detectable BSA or FBS protein binding. In contrast, the base polymers, without surfactant in the blend, readily bound the protein.

In addition, blends with HLB numbers of greater than 10 showed significant protein binding and in some cases, exhibited higher protein binding than the base polymers alone.

The blends were compounded on a Leistritz 34-mm twin screw extruder, zones set at 200° - 240°C, with a feed rate of 4.5 - 22.7 kg (10 - 50 pounds) per hour. Injection molding of the blends into 35mm petri dishes was performed on a 22.7 tonne (25 ton) Battenfeld molding machine, zones set at 200° - 250°C.

**Table**

| **Blended Molecule** | **Base Polymer** | **HLB #** | **Bound Protein Absorbance (550 nm)** | |
|---|---|---|---|---|
| | | | **BSA** | **FBS** |
| None (control) | polypropylene | N/A | 0.092 | 0.140 |
| PEO(2) OLEYL ETHER | polypropylene | 4.9 | 0.000 | 0.000 |
| PEO(2) STEARYL ETHER | polypropylene | 4.9 | 0.000 | 0.000 |
| SORBITOL MONO-STEARATE | polypropylene | 4.7 | 0.000 | 0.000 |
| SORBITOL MONO-OLEATE | polypropylene | 4.3 | 0.000 | 0.000 |
| SORBITOL TRI-OLEATE | polypropylene | 1.8 | 0.000 | 0.000 |
| PEO/PPO Block Copolymer Pluronic^{®} L-121 | polypropylene | 1.0 | 0.029 | 0.144 |
| PEO(4) SORBITOL MONO-STEARATE | polypropylene | 9.6 | 0.016 | 0.057 |
| PEO(4) SORBITOL MONO-LAURATE | polypropylene | 13.3 | 0.060 | 0.056 |
| PEO(20) SORBITOL MONO-LAURATE | polypropylene | 16.7 | 0.153 | 0.270 |
| PEO/PPO Block Copolymer Pluronic^{®} F-127 | polypropylene | 18 | 0.202 | 0.250 |
| PEO/PPO Block Copolymer Pluronic^{®} F-68 | polypropylene | >24 | 0.094 | 0.358 |
| | | | | |
| None (control) | ethylene vinyl acetate | N/A | 0.305 | 0.532 |
| PEO(2) OLEYL ETHER | ethylene vinyl acetate | 4.9 | 0.000 | 0.000 |
| 1% PEO(2) OLEYL ETHER | ethylene vinyl acetate | 4.9 | 0.000 | 0.291 |
| SORBITOL MONO-OLEATE | ethylene vinyl acetate | 4.3 | 0.000 | 0.000 |
| 1% SORBITOL MONO-OLEATE | ethylene vinyl acetate | 4.3 | 0.147 | 0.371 |
| PEO/PPO Block Copolymer Pluronic^{®} L-121 | ethylene vinyl acetate | 1 | 0.000 | N/A |
| | | | | |
| none | polystyrene (control) | N/A | 0.215 | 0.138 |
| PEO(2) OLEYL ETHER | polystyrene | 4.9 | 0.007 | 0.015 |
| PEO(2) STEARYL ETHER | polystyrene | 4.9 | 0.008 | 0.009 |
| SORBITOL TRI-STEARATE | polystyrene | 2.1 | 0.005 | 0.008 |
| PEO/PPO Block Copolymer Pluronic^{®} F-127 | polystyrene | 18 | 0.250 | N/A |
| PEO/PPO Block Copolymer Pluronic^{®} F-68 | polystyrene | >24 | 0.094 | 0.358 |

## Claims

1. Use of a product made from a polymer blend comprising:
a) a non-water soluble non-ionic surfactant having a hydrophilic-lipophilic balance number which is less than or equal to 10 and having at least one hydrophilic element which can extend into aqueous solution selected from alkyl alcohol ethoxylates, alkyl ester ethoxylates, sorbitol alkyl esters, ethylene oxide/propylene oxide block co-polymers and polypropylene oxide; and
b) a matrix polymer, wherein said product is capable of reducing the ability of an organic material in an aqueous solution to bind to its surface, for the manufacture of labware.

2. Use according to claim 1 wherein said non-water soluble non-ionic surfactant is selected from a group including sorbitol mono-oleate, sorbitol tristearate, sorbitol trioleate, sorbitol monostearate, PEO stearyl ether, PEO oleyl ether, and PEO/PPO block copolymer.

3. Use according to claim 1 or 2 wherein said matrix polymer is selected from the group including ethylene vinyl acetate, polypropylene, polyolefin, polyvinylchloride, polystyrene, polystyrene-butadiene copolymer, polycarbonate, polyacrylate, polyamide, and copolymers thereof, polyurethane, polyester and copolymers thereof, and fluoropolymers.

4. Use according to any of claims 1 to 3, wherein the polymer blend is made by a method, comprising the steps of:
a) thoroughly blending a non-water soluble non-ionic surfactant with the matrix polymer into a blend;
b) melting said blend; and
c) exposing said blend to sheer conditions such that said non-water soluble non-ionic surfactant will move to the surface of the matrix polymer substrate through sheer.

5. Use according to claim 4, wherein said blend is exposed to sheer conditions through extrusion or injection molding processes.

## Patentansprüche

1. Verwendung eines Produkts, das aus einem Polymergemisch hergestellt wird, umfassend:
a) ein nicht wasserlösliches, nichtionogenes Tensid mit einer hydrophil-lipophilen Gleichgewichtszahl, die weniger als oder gleich 10 beträgt, und mit mindestens einem hydrophilen Element, das sich in eine wässrige Lösung erstrecken kann, ausgewählt aus Alkylalkoholethoxylaten, Alkylesterethoxylaten, Sorbitolalkylestern, Ethylenoxid/Propylenoxid-Blockcopolymeren und Polypropylenoxid; und
b) ein Matrixpolymer, wobei das Produkt die Fähigkeit eines organischen Materials in einer wässrigen Lösung, an seine Oberfläche zu binden, mindern kann, zur Herstellung von Laborwaren.

2. Verwendung nach Anspruch 1, wobei das nicht wasserlösliche, nichtionogene Tensid aus einer Gruppe ausgewählt ist, die Sorbitmonooleat, Sorbittristearat, Sorbittrioleat, Sorbitmonostearat, PEO-Stearylether, PEO-Oleylether und PEO/PPO-Blockcopolymer beinhaltet.

3. Verwendung nach Anspruch 1 oder 2, wobei das Matrixpolymer aus der Gruppe ausgewählt ist, die Ethylenvinylacetat, Polypropylen, Polyolefin, Polyvinylchlorid, Polystyrol, Polystyrol/Butadien-Copolymer, Polycarbonat, Polyacrylat, Polyamid und Copolymere davon, Polyurethan, Polyester und Copolymere davon und Fluorpolymere beinhaltet.

4. Verwendung nach einem der Ansprüche 1 bis 3, wobei das Polymergemisch mit einem Verfahren hergestellt wird, das die folgenden Schritte umfasst:
a) gründliches Vermischen des nicht wasserlöslichen, nichtionogenen Tensids mit dem Matrixpolymer zu einem Gemisch;
b) Schmelzen des Gemischs und
c) Aussetzen des Gemischs gegenüber Scherbedingungen, so dass das nicht wasserlösliche, nichtionogene Tensid sich durch Scherung zur Oberfläche des Matrixpolymersubstrats bewegen wird.

5. Verwendung nach Anspruch 4, wobei das Gemisch mittels Extrusions- oder Spritzgussverfahren Scherbedingungen ausgesetzt wird.

## Revendications

1. Utilisation d'un produit fabriqué à partir d'un mélange de polymères comprenant :
a) un tensioactif non ionique non soluble dans l'eau ayant un rapport hydrolipophile qui est inférieur ou égal à 10 et ayant au moins un élément hydrophile qui peut être expansé dans une solution aqueuse, choisi parmi les éthoxylates d'alcool alkylique, les éthoxylates d'ester alkylique, les esters alkyliques de sorbitol, les copolymères séquencés oxyde d'éthylène/oxyde de propylène et l'oxyde de polypropylène ; et
b) un polymère de matrice, ledit produit étant capable de réduire la capacité d'un matériau organique dans une solution aqueuse à se lier à sa surface, pour la fabrication de verrerie de laboratoire.

2. Utilisation selon la revendication 1 dans laquelle ledit tensioactif non ionique non soluble dans l'eau est choisi dans un groupe incluant un mono-oléate de sorbitol, un tristéarate de sorbitol, un trioléate de sorbitol, un monostéarate de sorbitol, un éther stéarylique de PEO, un éther oléylique de PEO et un copolymère séquencé PEO/PPO.

3. Utilisation selon la revendication 1 ou 2 dans laquelle ledit polymère de matrice est choisi dans le groupe incluant le vinylacétate d'éthylène, le polypropylène, une polyoléfine, le polychlorure de vinyle, le polystyrène, un copolymère polystyrène-butadiène, le polycarbonate, le polyacrylate, le polyamide, et les copolymères de ceux-ci, le polyuréthane, le polyester et les copolymères de ceux-ci et les fluoropolymères.

4. Utilisation selon l'une quelconque des revendications 1 à 3 dans laquelle le mélange de polymères est fabriqué par un procédé comprenant les étapes suivantes :
a) mélange intime d'un tensioactif non ionique non soluble dans l'eau avec le polymère de matrice pour former un mélange ;
b) fusion dudit mélange ; et
c) exposition dudit mélange à des conditions de cisaillement telles que ledit tensioactif non ionique non soluble dans l'eau migre vers la surface du substrat du polymère de matrice par cisaillement.

5. Utilisation selon la revendication 4, dans laquelle ledit mélange est exposé à des conditions de cisaillement par des procédés d'extrusion ou de moulage par injection.
